# EUROPEAN PATENT APPLICATION

(11) **EP 2 163 191 A1**
(43) Date of publication of application: **17.03.2010**
(21) Application number: 09011445.5
(22) Date of filing: 07.09.2009
(51) Int. Cl.: A61B 5/00

(54) **Diagnostic imaging apparatus**

(30) Priority: 16.09.2008 JP 2008236979
(71) Applicant: Fujifilm Corporation, Tokyo 106-8620 (JP)
(72) Inventor: Watanabe, Daisuke, Ashigarakami-gun, Kanagawa 258-8538 (JP); Kinjo, Naoto, Ashigarakami-gun, Kanagawa 258-8538 (JP)
(74) Representative: Klunker . Schmitt-Nilson . Hirsch

(57) **Abstract**

A diagnosis apparatus (10) in which a lesion in the large intestine can be diagnosed with high precision by detecting pits is provided. A three-dimensional optical tomographic image is obtained by the configuration of an apparatus using an endoscope (100) and an optical probe, images in X-Y planes perpendicular to the depth direction of a living body tissue are cut out at a plurality of depth positions, based on the three-dimensional tomographic image data, and a pit pattern shape highlighted image is generated from their average image to perform diagnostic support.

## Description

### BACKGROUND OF THE INVENTION

### Field of the Invention

The present invention relates to an electronic endoscope apparatus and an OCT apparatus and to a diagnostic imaging apparatus in which three-dimensional volume data is obtained to perform the diagnosis of a lesion in the large intestine.

### Description of the Related Art

Conventionally, as an endoscope apparatus for observing the inside of the body cavity of a living body, electronic endoscope apparatuses, in which illumination light illuminates in the body cavity of a living body, and an image formed by reflected light is picked up and displayed on a monitor or the like, have been widely spread and used in various fields.

Also, as in Japanese Patent Application Laid-Open No. 2002-148185, many endoscope apparatuses comprise a forceps port, and by a probe introduced into the body cavity through this forceps port, the biopsy and treatment of a tissue in the body cavity can be performed.

In recent years, the development of a tomographic image obtaining apparatus in which a tomographic image of a living body or the like is obtained without cutting an object to be measured, such as a living body tissue, has been in progress. For example, an optical tomographic imaging apparatus using optical coherence tomography (OCT) measurement using the coherence of low coherence light is known.

In this optical tomographic imaging apparatus using the OCT measurement, low coherence light emitted from a light source comprising an SLD (super luminescent diode) and the like is divided into signal light and reference light, the frequency of the reference light or the signal light is slightly shifted by a piezoelectric element or the like, the signal light enters a measurement portion so that return light reflected at a predetermined depth of the measurement portion and the reference light interfere with each other, and the light intensity of the coherent light is measured by heterodyne detection to obtain tomographic information.

According to this optical tomographic imaging apparatus, by slightly moving a movable mirror and the like located in the optical path of the reference light to slightly change the optical path length of the reference light, information at the depth of the measurement portion where the optical path length of the reference light and the optical path length of the signal light match can be obtained. Also, by repeating measurement, while slightly displacing the signal light incidence point, an optical tomographic image in a predetermined scan region can be obtained. Further, by displacing the signal light incidence point in a direction perpendicular to the tomographic plane to obtain a plurality of optical tomographic images, three-dimensional image volume data can also be obtained.

In such an OCT apparatus (optical tomographic imaging apparatus), a measurement site can be finely (a resolution of about 10 µm) observed, and by inserting an OCT probe (optical probe) into the forceps port of an endoscope apparatus to guide signal light and the return light of the signal light from a living body to obtain an optical tomographic image in the body cavity, for example, the invasion depth diagnosis of early cancer is also possible.

Also, in recent years, large intestine cancer has tended to increase, and an improvement in the precision of large intestine lesion diagnosis has been desired. It has been clear that it is effective to observe the shape of large intestine pits in the large intestine lesion diagnosis ("Large Intestine Pit Pattern Diagnosis" written and edited by Shinei Kudo).

However, for the observed image of an endoscope by normal light, mainly, a living body tissue surface can be observed, but observation is not possible up to the inside of the tissue, and it is difficult to detect a large intestine pit lesion inside the tissue, particularly in a deep part.

Also, in biopsy, there is also a method for performing examination by taking a mucosa tissue and photographing a cross section by a microscope. But, the processing of cutting the tissue can be finely performed only at intervals at a level of up to 5 mm, and precise diagnosis at the µm level is impossible.

### SUMMARY OF THE INVENTION

The present invention has been made in view of such circumstances, and it is an object of the present invention to provide a diagnosis apparatus in which the abnormality of large intestine pits in the mucosa layer of the large intestine (from the surface of the large intestine to at least the deep part of the muscularis mucosae) can be diagnosed by an observed image.

To achieve the above object, a diagnostic imaging apparatus according to the first aspect of the present invention, comprises:
a three-dimensional optical tomographic image generating device in which a three-dimensional optical tomographic image is generated, based on return light from a living body obtained by an optical probe inserted into a body to be measured from a forceps port of an endoscope,
a setting device in which a Z-axis is set as an axis in a depth direction generally perpendicular to a depth direction of a living body tissue, based on the three-dimensional optical tomographic image data, and an X-axis and a Y-axis are set in a plane perpendicular to the Z-axis, and
a cut out images generating device in which cut out images obtained by cutting out images in X-Y planes at a plurality of levels of depth are generated, so that diagnostic support is performed using the plurality of cut out images.

Also, as shown in the second aspect of the present invention, an average image is created by average calculation of pixels in the same coordinates in the X-Y planes, using the plurality of cut out images cut out at the plurality of depths, and display is performed so that a pit pattern corresponding to a large intestine pit can be observed in the average image.

Also, as shown in the third aspect of the present invention, a pixel having a predetermined condition is highlighted in the average image. Thus, it is also possible to increase effect.

Also, as shown in the fourth aspect of the present invention, the cut out images cut out at the plurality of depths are located so that the cut out images can be compared and observed on the same screen, and display is performed so that a pit pattern corresponding to a large intestine pit can be observed.

Also here, it is also possible to increase effect by highlighting a pixel having a predetermined condition in the cut out images, as shown in the fifth aspect of the present invention. It is desired that the difference between the images is highlighted.

Further, it is possible not only to highlight the images in the X-Y planes and average image of the tomographic images, but also to provide an automatic diagnosis function, as follows.

As shown in the sixth aspect of the present invention, a normal large intestine pit pattern is set as a reference pattern, a pit pattern is extracted from one of the cut out images and the average image, similarity between the extracted pit pattern and the reference pattern is derived, an automatic diagnostic point is derived by summation of the similarity for each block which divides an area in an observation field of view into a plurality of areas, and a distribution of the automatic diagnostic points in the observation field of view is displayed.

Thus, diagnostic support can be performed so that a suspicion of a large intestine lesion is easily visually determined.

According to the present invention, the shape of large intestine pits can be observed up to the deep part of the tissue throughout the large intestine mucosa layer. Particularly, it is possible to cut out a plurality of images in the depth direction of the tissue, corresponding to the large intestine pit structure, and highlight the large intestine pit structure, compared with noises and other tissue structures, by the state of change in the images and an average image, and it is possible to perform the diagnosis of a large intestine lesion with high precision.

### BRIEF DESCRIPTION OF THE DRAWINGS

Fig. 1 is an appearance view showing a diagnostic imaging apparatus 10 according to the present invention;
Fig. 2 is a block diagram showing the inner configuration of an OCT processor 400 and an OCT probe 600;
Fig. 3 is a cross-sectional view of the OCT probe 600;
Fig. 4 is a view showing a state in which a tomographic image is obtained using the OCT probe 600 led out from the forceps port 156 of an endoscope 100;
Figs. 5A and 5B are views showing volume data and a three-dimensional image generated based on the volume data;
Fig. 6 is an appearance view of a probe outer tube 620 in this embodiment;
Fig. 7 is a flow chart showing the generation of three-dimensional volume data using the probe outer tube 620 with markers;
Figs. 8A and 8B are views showing an optical tomographic image obtained using the probe outer tube 620 with markers;
Fig. 9 is a view showing that the optical tomographic images are rotated to match the phase of the marker portion 648;
Fig. 10 is a view showing a state in which the images 640 are extracted;
Figs. 11A and 11B are views showing one example of the movement variations correction marker 630 and positions where the image(s) 640 and the images 642 are obtained;
Figs. 12A and 12B are views showing one example of a three-dimensional tomographic image of a large intestine mucosa;
Figs. 13A, 13B, and 13C are views showing one example in which images in X-Y planes are cut out from a three-dimensional tomographic image in a pit in a normal portion, and an average image in the X-Y plane is created;
Figs. 14A, 14B, and 14C are views showing one example in which images in X-Y planes are cut out from a three-dimensional tomographic image in a pit in a lesion, and an average image in the X-Y plane is created;
Fig. 15 is a view showing an example of a display image during diagnosis in which an average image in the X-Y plane is displayed from a three-dimensional tomographic image;
Fig. 16 is a view showing a distribution of automatic diagnostic points in a lesion in an average image in the X-Y plane; and
Fig. 17 is a flow chart showing processing in a second embodiment.

### DETAILED DESCRIPTION OF THE PREFERRED EMBODIMENTS

Best modes for carrying out the present invention will be described below.

### <Appearance of Diagnostic Imaging Apparatus>

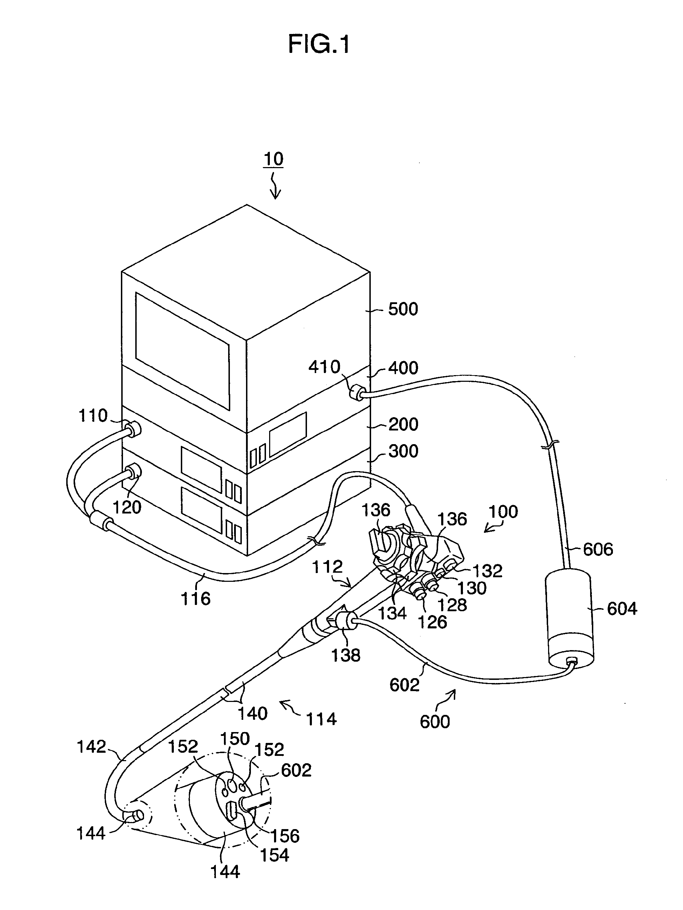

Fig. 1 is an appearance view showing a diagnostic imaging apparatus 10 according to the present invention.

As shown in the figure, this diagnostic imaging apparatus 10 is mainly composed of an endoscope 100, an endoscope processor 200, a light source apparatus 300, an OCT processor 400, and a monitor apparatus 500. The endoscope processor 200 may be configured to contain the light source apparatus 300.

The endoscope 100 comprises a hand operation portion 112 and an insertion portion 114 provided continuously to this hand operation portion 112. An operator grasps and operates the hand operation portion 112, and inserts the insertion portion 114 into the body of a test subject to perform observation.

A forceps insertion portion 138 is provided in the hand operation portion 112, and this forceps insertion portion 138 is in communication with the forceps port 156 of a tip portion 144. In the diagnostic imaging apparatus 10 according to the present invention, by inserting an OCT probe 600 from the forceps insertion portion 138, the OCT probe 600 is led out from the forceps port 156. The OCT probe 600 is composed of an insertion portion 602 which is inserted from the forceps insertion portion 138 and led out from the forceps port 156, and an operation portion 604 for the operator to operate the OCT probe 600, and a cable 606 connected to the OCT processor 400 via a connector 410.

### <Configuration of Endoscope, Endoscope Processor, and Light Source Apparatus>

### [Endoscope]

An observation optical system 150, illumination optical systems 152, and a CCD 180 are disposed in the tip portion 144 of the endoscope 100.

The observation optical system 150 images a test body on the light receiving surface of a CCD not shown, and the CCD converts an image of the test body, which is imaged on the light receiving surface, to an electrical signal by each light receiving element. The CCD in this embodiment is a color CCD in which color filters of three primary colors, red (R), green (G), and blue (B), are disposed in predetermined arrangement (Bayer arrangement or honeycomb arrangement) for each pixel.

### [Light Source Apparatus]

The light source apparatus 300 allows visible light to enter a light guide not shown. One end of the light guide is connected to the light source apparatus 300 via an LG connector 120, and the other end of the light guide faces the illumination optical systems 152. Light emitted from the light source apparatus 300 is emitted from the illumination optical systems 152 via the light guide and illuminates the field of view of the observation optical system 150.

### [Endoscope Processor]

An image signal output from the CCD is input to the endoscope processor 200 via an electrical connector 110. This analog image signal is converted to a digital image signal in the endoscope processor 200 and subjected to necessary processing to be displayed on the screen of the monitor apparatus 500.

Also, the image signal of a tomographic image output from the OCT processor 400 is input to the endoscope processor 200. The endoscope processor 200 generates three-dimensional volume data, based on the image signals of a plurality of tomographic images. The generated three-dimensional image is also subjected to necessary processing and output to the monitor apparatus 500.

In this manner, the data of an observed image obtained by the endoscope 100 is output to the endoscope processor 200, and an image is displayed on the monitor apparatus 500 connected to the endoscope processor 200.

### <Inner Configuration of OCT Processor and OCT Probe>

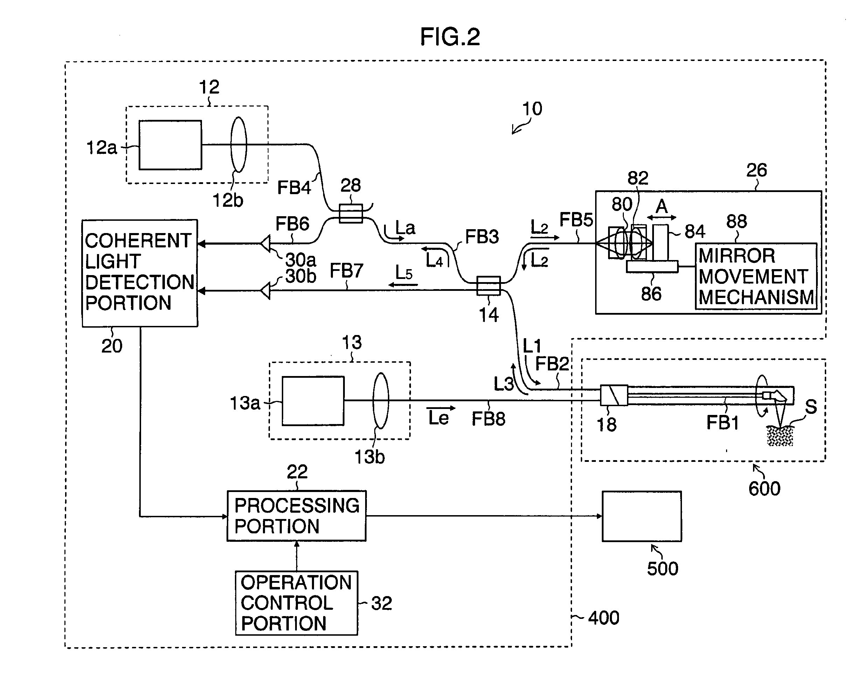

Fig. 2 is a block diagram showing the inner configuration of the OCT processor 400 and the OCT probe 600.

### [OCT Processor]

The OCT processor 400 and the OCT probe 600 shown in Fig. 2 are to obtain an optical tomographic image of an object to be measured, by optical coherence tomography (OCT) measurement, and have a first light source (first light source unit) 12 which emits light La for measurement, an optical fiber coupler (dividing and combining portion) 14 which divides the light La, emitted from the first light source 12, into measurement light (first light beam) L1 and reference light L2 and combines return light L3 from an object to be measured S, a test body, and the reference light L2 to generate coherent light L4, an optical probe 16 comprising a rotation side optical fiber FB1 which guides the measurement light L1, divided by the optical fiber coupler 14, to the object to be measured and guides the return light L3 from the object to be measured, a fixed side optical fiber FB2 which guides the measurement light L1 to the rotation side optical fiber FB1 and guides the return light L3 guided by the rotation side optical fiber FB1, an optical connector 18 which rotatably connects the rotation side optical fiber FB to the fixed side optical fiber FB2 and transmits the measurement light L1 and the return light L3, a coherent light detection portion 20 which detects as a coherent signal the coherent light L4 generated by the optical fiber coupler 14, and a processing portion 22 which processes the coherent signal detected by this coherent light detection portion 20 to obtain an optical tomographic image (hereinafter also simply referred to as a "tomographic image"). Also, the optical tomographic image obtained by the processing portion 22 is displayed on the monitor apparatus 500.

Also, the OCT processor 400 has a second light source (second light source unit) 13 which emits aiming light (second light beam) Le for indicating a measurement mark, an optical path length adjustment portion 26 which adjusts the optical path length of the reference light L2, an optical fiber coupler 28 which separates the light La emitted from the first light source 12, detection portions 30a and 30b which detect the return light L4 and return light L5 combined by the optical fiber coupler 14, and an operation control portion 32 which performs the input of various conditions to the processing portion 22, and the change of setting, and the like.

In the OCT processor 400 shown in Fig. 2, various optical fibers FB (FB3, FB4, FB5, FB6, FB7, FB8, and the like), including the rotation side optical fiber FB1 and the fixed side optical fiber FB2, are used as light paths for guiding and transmitting various lights, including the above-described emitted light La, aiming light Le, measurement light L1, reference light L2, and return light L3, and the like, between components, such as optical devices.

The first light source 12 emits light for OCT measurement (for example, laser light having a wavelength of 1.3 µm or low coherence light) and comprises a light source 12a which emits laser light or low coherence light La, and a lens 12b which collects the light La emitted from the light source 12a. As described in detail later, the light La emitted from the first light source 12 is divided into the measurement light L1 and the reference light L2 by the optical fiber coupler 14 via the optical fibers FB4 and FB3, and the measurement light L1 is input to the optical connector 18.

Also, the second light source 13 emits visible light as the aiming light Le so that a measurement site is easily checked. For example, red semiconductor laser light having a wavelength of 0.66 µm, He-Ne laser light having a wavelength of 0.63 µm, blue semiconductor laser light having a wavelength of 0.405 µm, and the like can be used. The second light source 13 comprises, for example, a semiconductor laser 13a which emits red, blue, or green laser light, and a lens 13b which collects the aiming light Le emitted from the semiconductor laser 13a. The aiming light Le emitted from the second light source 13 is input to the optical connector 18 via the optical fiber FB8.

In the optical connector 18, the measurement light L1 and the aiming light Le are combined and guided to the rotation side optical fiber FB 1 in the optical probe 16.

The optical fiber coupler (dividing and combining portion) 14 is composed of, for example, a 2 x 2 optical fiber coupler, and is optically connected to each of the fixed side optical fiber FB2, the optical fiber FB3, the optical fiber FB5, and the optical fiber FB7.

The optical fiber coupler 14 divides the light La, which enters from the first light source 12 via the optical fibers FB4 and FB3, into the measurement light (first light beam) L1 and the reference light L2, allows the measurement light L1 to enter the fixed side optical fiber FB2, and allows the reference light L2 to enter the optical fiber FB5.

Further, the optical fiber coupler 14 combines the light L2, which enters the optical fiber FB5, is subjected to frequency shift and the change of the optical path length by the optical path length adjustment portion 26 described later, and returned to the optical fiber FB5, and the light L3, which is obtained by the optical probe 16 described later, and guided from the fixed side optical fiber FB2, and emits the combined light to the optical fiber FB3 (FB6) and the optical fiber FB7.

The optical probe 16 is connected to the fixed side optical fiber FB2 via the optical connector 18, and the measurement light L1 combined with the aiming light Le enters the rotation side optical fiber FB 1 from the fixed side optical fiber FB2 via the optical connector 18. This entering measurement light L1 combined with the aiming light Le is transmitted by the rotation side optical fiber FB1 and illuminates the object to be measured S. Then, the return light L3 from the object to be measured S is obtained, and the obtained return light L3 is transmitted by the rotation side optical fiber FB 1 and emitted to the fixed side optical fiber FB2 via the optical connector 18.

The optical connector 18 combines the measurement light (first light beam) L1 and the aiming light (second light beam) Le.

The coherent light detection portion 20 is connected to the optical fiber FB6 and the optical fiber FB7 and detects as coherent signals the coherent light L4 and the coherent light L5 generated by combining the reference light L2 and the return light L3 by the optical fiber coupler 14.

Here, the OCT processor 400 has a detector 30a which is provided on the optical fiber FB6 branched from the optical fiber coupler 28 and detects the light intensity of the laser light L4, and a detector 30b which detects the light intensity of the coherent light L5 on the optical path of the optical fiber FB7.

The coherent light detection portion 20 extracts only coherent amplitude components from the coherent light L4 detected from the optical fiber FB6, and the coherent light L5 detected from the optical fiber FB7, based on the detection results of the detector 30a and the detector 30b.

The processing portion 22 detects, from the coherent signals extracted by the coherent light detection portion 20, a region where the optical probe 16 at a measurement position and the object to be measured S are in contact with each other, more accurately, a region where the surface of the probe outer tube (described later) of the optical probe 16 and the surface of the object to be measured S are considered to be in contact with each other. Further, the processing portion 22 obtains a tomographic image from the coherent signals detected by the coherent light detection portion 20, and outputs the obtained tomographic image to the endoscope processor 200.

The optical path length adjustment portion 26 is located on the reference light L2 emission side of the optical fiber FB5 (that is, the end of the optical fiber FB5 opposite to the optical fiber coupler 14).

The optical path length adjustment portion 26 has a first optical lens 80 which turns light, emitted from the optical fiber FB5, into parallel light, a second optical lens 82 which collects the light turned into the parallel light by the first optical lens 80, a reflecting mirror 84 which reflects the light collected by the second optical lens 82, a base 86 which supports the second optical lens 82 and the reflecting mirror 84, and a mirror movement mechanism 88 which moves the base 86 in a direction parallel to the direction of the optical axis. The optical path length adjustment portion 26 adjusts the optical path length of the reference light L2 by changing the distance between the first optical lens 80 and the second optical lens 82.

The first optical lens 80 turns the reference light L2, which is emitted from the core of the optical fiber FB5, into parallel light, and collects the reference light L2, which is reflected by the reflecting mirror 84, in the core of the optical fiber FB5.

Also, the second optical lens 82 collects the reference light L2, which is turned into parallel light by the first optical lens 80, on the reflecting mirror 84, and turns the reference light L2, which is reflected by the reflecting mirror 84, into parallel light. In this manner, a confocal optical system is formed by the first optical lens 80 and the second optical lens 82.

Further, the reflecting mirror 84 is located at the focus of the light collected by the second optical lens 82 and reflects the reference light L2 collected by the second optical lens 82.

Thus, the reference light L2 emitted from the optical fiber FB5 is turned into parallel light by the first optical lens 80 and collected on the reflecting mirror 84 by the second optical lens 82. Then, the reference light L2 reflected by the reflecting mirror 84 is turned into parallel light by the second optical lens 82 and collected in the core of the optical fiber FB5 by the first optical lens 80.

Also, the second optical lens 82 and the reflecting mirror 84 are fixed to the base 86, and the mirror movement mechanism 88 moves the base 86 in the direction of the optical axis of the first optical lens 80 (the direction of the arrow A in Fig. 2).

By moving the base 86 in the direction of the arrow A by the mirror movement mechanism 88, the distance between the first optical lens 80 and the second optical lens 82 can be changed, so that the optical path length of the reference light L2 can be adjusted.

The operation control portion 32 has an input device, such as a keyboard and a mouse, and a control device which controls various conditions based on input information. The operation control portion 32 is connected to the processing portion 22. The operation control portion 32 performs the input, setting, change, and the like of various processing conditions and the like in the processing portion 22, based on the instruction of an operator input from the input device.

The operation control portion 32 may display an operation screen on the monitor apparatus 500 and may display an operation screen on a display portion separately provided. Also, operation control and the setting of various conditions for the first light source 12, the second light source 13, the optical connector 18, the coherent light detection portion 20, the optical path length, and the detection portions 30a and 30b may be performed by the operation control portion 32.

### [OCT Probe]

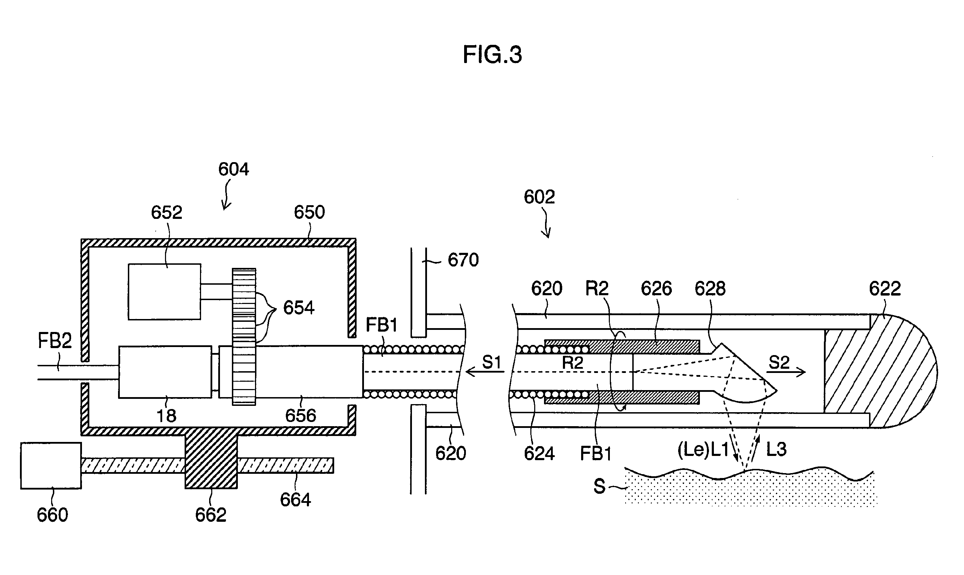

Fig. 3 is a cross-sectional view of the OCT probe 600.

As shown in Fig. 3, the tip portion of the insertion portion 602 has a probe outer tube 620, a cap 622, the rotation side optical fiber FB1, a spring 624, a fixing member 626, and an optical lens 628.

The probe outer tube (sheath) 620 is a tubular member having flexibility and is made of a material that transmits the measurement light L1, which is combined with the aiming light Le in the optical connector 18, and the return light L3. In the probe outer tube 620, part of the side of the tip through which the measurement light L1 (aiming light Le) and the return light L3 pass (the tip of the rotation side optical fiber FB1 opposite to the optical connector 18, hereinafter referred to as the tip of the probe outer tube 620) should be formed of a material that transmits light (a transparent material), around the entire periphery, and portions other than the tip may be formed of a material that does not transmit light.

The cap 622 is provided at the tip of the probe outer tube 620 and closes the tip of the probe outer tube 620.

The rotation side optical fiber FB 1 is a linear member and is housed in the probe outer tube 620 along the probe outer tube 620. The rotation side optical fiber FB 1 guides the measurement light L1, which is emitted from the fixed side optical fiber FB2 and combined by the optical connector 18 with the aiming light Le emitted from the optical fiber FB8, to the optical lens 628, guides the return light L3 from the object to be measured S, which is obtained by the optical lens 628 by illuminating the object to be measured S with the measurement light L1 (aiming light Le), to the optical connector 18, and allows the return light L3 to enter the fixed side optical fiber FB2.

Here, the rotation side optical fiber FB1 and the fixed side optical fiber FB2 are connected by the optical connector 18, and are optically connected, with the rotation of the rotation side optical fiber FB1 not transferred to the fixed side optical fiber FB2. Also, the rotation side optical fiber FB1 is located rotatably with respect to the probe outer tube 620 and movably in the axial direction of the probe outer tube 620.

The spring 624 is fixed on the outer periphery of the rotation side optical fiber FB 1. Also, the rotation side optical fiber FB1 and the spring 624 are connected to the optical connector 18.

The optical lens 628 is located at the tip on the measurement side of the rotation side optical fiber FB1 (the tip of the rotation side optical fiber FB1 opposite to the optical connector 18), and its tip portion is formed in a generally spherical shape to collect the measurement light L1 (the aiming light Le), which is emitted from the rotation side optical fiber FB1, on the object to be measured S.

The optical lens 628 illuminates the object to be measured S with the measurement light L1 (aiming light Le) emitted from the rotation side optical fiber FB1, collects the return light L3 from the object to be measured S, and allows the return light L3 to enter the rotation side optical fiber FB1.

The fixing member 626 is located on the outer periphery of the connection portion between the rotation side optical fiber FB 1 and the optical lens 628 and fixes the optical lens 628 to the end of the rotation side optical fiber FB 1. Here, the method for fixing the rotation side optical fiber FB1 and the optical lens 628 by the fixing member 626 is not particularly limited, and the fixing member 626 and the rotation side optical fiber FB1 and the optical lens 628 may be adhered and fixed with an adhesive or may be fixed by a mechanical structure, using a bolt or the like. For the fixing member 626, any may be used as long as it is one used for the fixing, holding, or protection of the optical fiber, for example, a zirconia ferrule and a metal ferrule.

Also, the rotation side optical fiber FB1 and the spring 624 are connected to a rotating tube 656 described later. By rotating the rotation side optical fiber FB1 and the spring 624 by the rotating tube 656, the optical lens 628 is rotated in the direction of the arrow R2 with respect to the probe outer tube 620. Also, the optical connector 18 comprises a rotation encoder, and the position of the illumination of the measurement light L1 is detected from information on the position (information on the angle) of the optical lens 628, based on a signal from the rotation encoder. In other words, the measurement position is detected by detecting the angle of the rotating optical lens 628 to the reference position in the direction of rotation.

Further, the rotation side optical fiber FB1, the spring 624, the fixing member 626, and the optical lens 628 are configured to be movable inside the probe outer tube 620 in the direction of the arrow S 1 (the direction of the forceps port) and the direction of S2 (the direction of the tip of the probe outer tube 620) by a drive portion described later.

Also, a view showing a schematic of the drive portion for the rotation side optical fiber FB1 and the like in the operation portion 604 of the OCT probe 600 is on the left side of Fig. 3.

The probe outer tube 620 is fixed to a fixing member 670. On the other hand, the rotation side optical fiber FB 1 and the spring 624 are connected to the rotating tube 656, and the rotating tube 656 is configured to rotate via a gear 654 according to the rotation of a motor 652. The rotating tube 656 is connected to the optical connector 18, and the measurement light L1 and the return light L3 are transmitted between the rotation side optical fiber FB1 and the fixed side optical fiber FB2 via the optical connector 18.

Also, a frame 650 containing these comprises a support member 662, and the support member 662 has a screw hole not shown. A ball screw for back and forth movement 664 is engaged with the screw hole, and a motor 660 is connected to the ball screw for back and forth movement 664. Therefore, by rotation-driving the motor 660, the frame 650 is moved back and forth, thereby, the rotation side optical fiber FB1, the spring 624, the fixing member 626, and the optical lens 628 can be moved in the S1 and S2 directions in Fig. 3.

The OCT probe 600 has a configuration as described above, and by rotating the rotation side optical fiber FB1 and the spring 624 in the direction of the arrow R2 in Fig. 3 by the optical connector 18, the measurement light L1 (aiming light Le) emitted from the optical lens 628 illuminates the object to be measured S, while scanning is performed in the direction of the arrow R2 (the circumferential direction of the probe outer tube 620), to obtain the return light L3. The aiming light Le illuminates the object to be measured S, for example, as blue, red, or green spot light, and the reflected light of this aiming light Le is also displayed as a bright point in an observed image displayed on the monitor apparatus 500.

Thus, around the entire periphery of the probe outer tube 620 in the circumferential direction, the desired site of the object to be measured S can be accurately captured, and the return light L3 reflected from the object to be measured S can be obtained.

Further, when a plurality of tomographic images for generating three-dimensional volume data are obtained, the optical lens 628 is moved to an end in a movable range in the direction of the arrow S1 by the drive portion, and moved in the S2 direction by a predetermined amount at a time, while a tomographic image is obtained, or moved to an end in the movable range, while obtaining a tomographic image and movement in the S2 direction by a predetermined amount are alternately repeated.

In this manner, a plurality of tomographic images in the desired range for the object to be measured S are obtained, and based on the plurality of tomographic images obtained, three-dimensional volume data can be obtained.

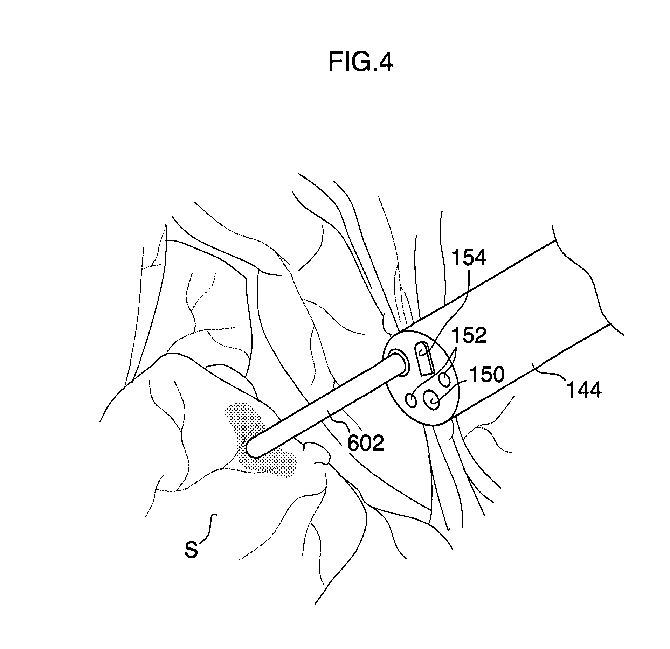

Fig. 4 is a view showing a state in which a tomographic image is obtained using the OCT probe 600 led out from the forceps port 156 of the endoscope 100. As shown in the figure, the tip portion of the insertion portion 602 of the OCT probe is brought near the desired site of the object to be measured S to obtain a tomographic image. When a plurality of tomographic images in the desired range are obtained, the main body of the OCT probe 600 need not be moved, and the optical lens 628 should be moved in the probe outer tube 620 by the above-described drive portion.

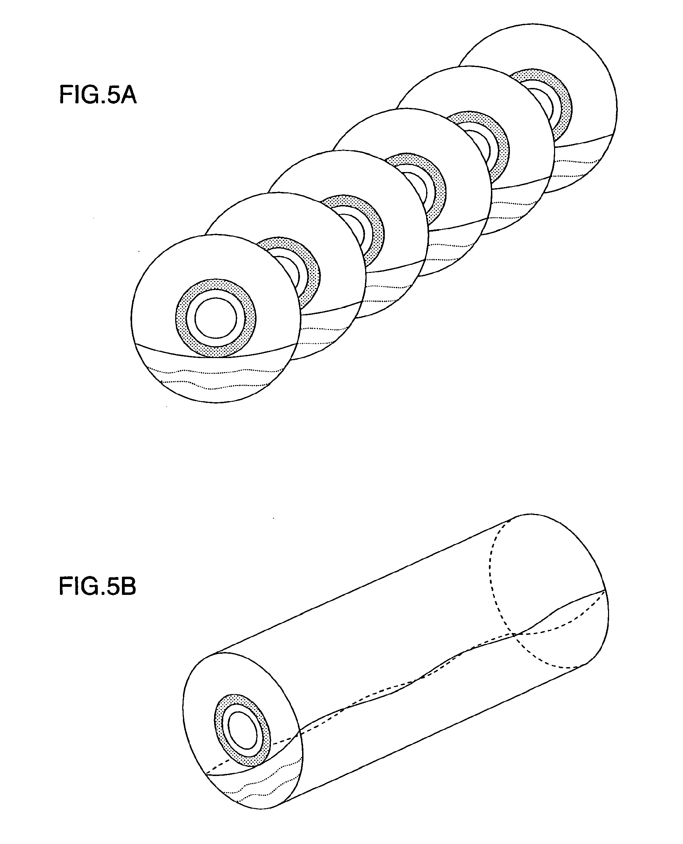

Fig. 5A shows three-dimensional volume data in which a plurality of tomographic images obtained by alternately performing the movement of the optical lens 628 of the OCT probe 600 by a predetermined amount and obtaining an optical tomographic image are arrayed. Also, Fig. 5B shows a three-dimensional image generated based on the three-dimensional volume data shown in Fig. 5A. In this manner, by moving the optical lens 628 to obtain a plurality of tomographic images, and performing image processing, three-dimensional volume data can be generated.

Here, the three-dimensional image is generated from the three-dimensional volume data, assuming that adjacent tomographic images in the volume data are obtained at equal intervals. However, in the mechanism in which the frame 650 is moved back and forth by rotation-driving the motor 660, as shown in Fig. 4, operation variations, such as the rotation variations of the motor 660, movement variations caused by machine precision and the like, and movement variations caused by the thermal expansion of each portion due to temperature, occur, so that the interval at which the tomographic images are obtained may not be equal.

### <Operation of Diagnostic Imaging Apparatus>

The diagnostic imaging apparatus 10 according to the present invention generates three-dimensional volume data without the effect of the operation variations of the drive portion by obtaining optical tomographic images using the probe outer tube 620 in which markers are provided, and performing image processing using the markers appearing in the optical tomographic images.

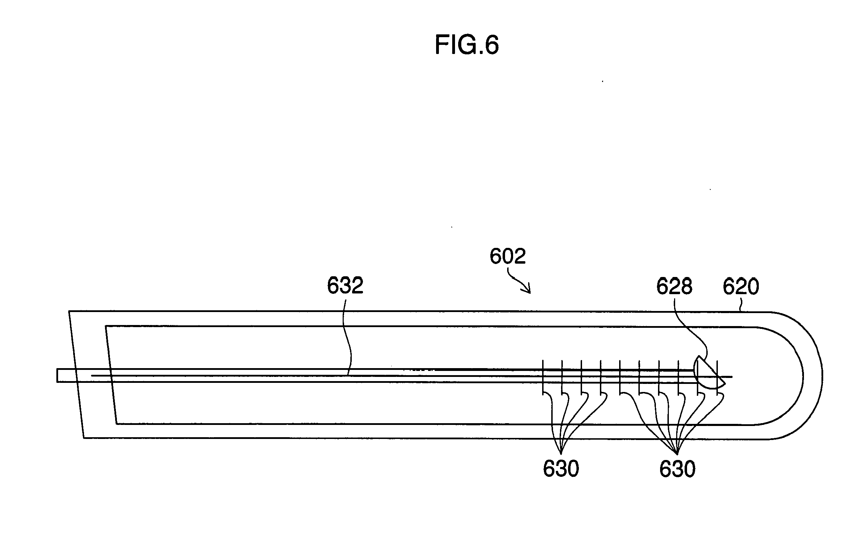

Fig. 6 is an appearance view of the probe outer tube 620 in this embodiment. As shown in the figure, rotation variations correction marker 632 which is drawn in a line parallel to the long axis direction of the probe outer tube 620, and movement variations correction markers 630 which are in part of the circumferential direction of the probe outer tube 620 orthogonal to the rotation variations correction marker 632 and are drawn at equal intervals in the long axis direction of the probe outer tube 620 are provided in the probe outer tube 620. The movement variations correction markers 630 and the rotation variations correction marker 632 are provided on the inner wall surface of the probe outer tube 620.

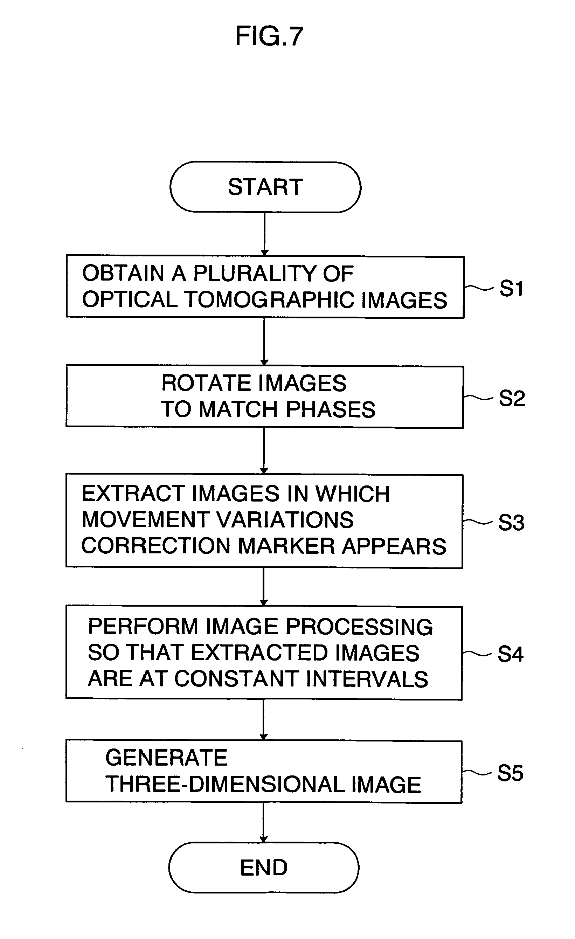

The processing of obtaining three-dimensional volume data using the probe outer tube 620 with markers shown in Fig. 6 will be described with reference to Fig. 7.

First, a plurality of optical tomographic images used for the generation of three-dimensional volume data are obtained (step S1). The operator sets the probe outer tube 620 so that the rotation variations correction marker 632 and the affected part of a test body for which tomographic images are desired to be obtained are 180 degrees opposite to each other around the circumference of the probe outer tube 620. To obtain the plurality of optical tomographic images, the optical lens 628 is moved to an end in a movable range in the direction of the arrow S 1 in Fig. 3 by the drive portion, and moved to an end in the movable range, while obtaining an optical tomographic image and movement in the S2 direction by a predetermined amount are alternately repeated, as described above.

Therefore, there are two types, an optical tomographic image obtained at a position where the movement variations correction marker 630 is present, and an optical tomographic image obtained at a position where the movement variations correction marker 630 is not present (a position where only the rotation variations correction marker 632 is present), in the plurality of optical tomographic images.

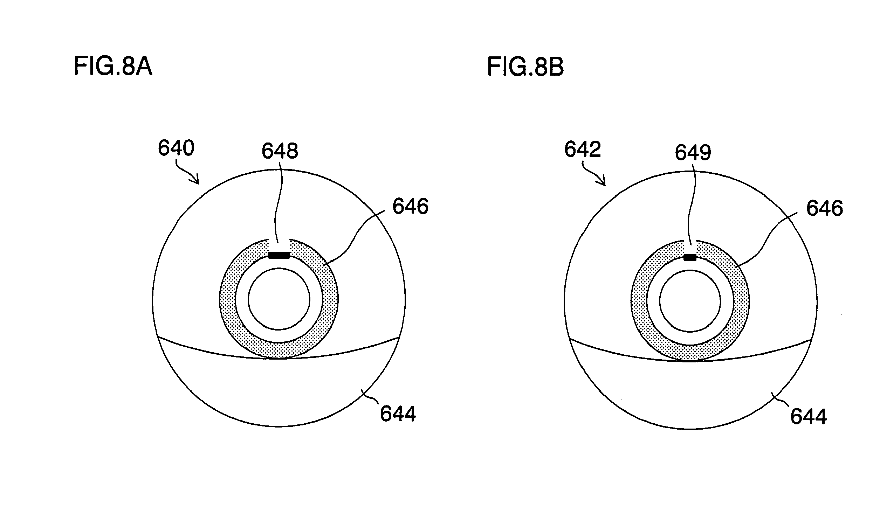

Figs. 8A and 8B are views showing optical tomographic images obtained using the probe outer tube 620 with markers shown in Fig. 6. Fig. 8A is a view showing an optical tomographic image 640 obtained at the position of the movement variations correction marker 630. In addition to a cross section of a test body 644, a cross section 646 of the probe outer tube 620 is in the optical tomographic image 640. Also, a portion where signal light is cut off by the movement variations correction marker 630 and an image is not extracted, that is, a portion 648 where the movement variations correction marker 630 appears, is present in the cross section 646 of the probe outer tube 620.

Also, Fig. 8B is a view showing an optical tomographic image 642 obtained at a position where the movement variations correction marker 630 is not present. In addition to a cross section of the test body 644, the cross section 646 of the probe outer tube 620 is also in the optical tomographic image 642. Also, a portion where signal light is cut off by the rotation variations correction marker 632 and an image is not extracted, that is, a portion 649 where the rotation variations correction marker 632 appears, is present in the cross section 646 of the probe outer tube 620. This marker portion 649 is different in length from the marker portion 648 of the movement variations correction marker 630.

For the plurality of optical tomographic images obtained in this manner, the phases of the optical tomographic images in the rotation direction may not match due to the rotation variations of the motor 652, and the like, as described above. The processing portion 22 of the OCT processor 400 rotates the respective optical tomographic images so that the centers of the marker portions 648 and 649 in the optical tomographic images are in phase (step S2).

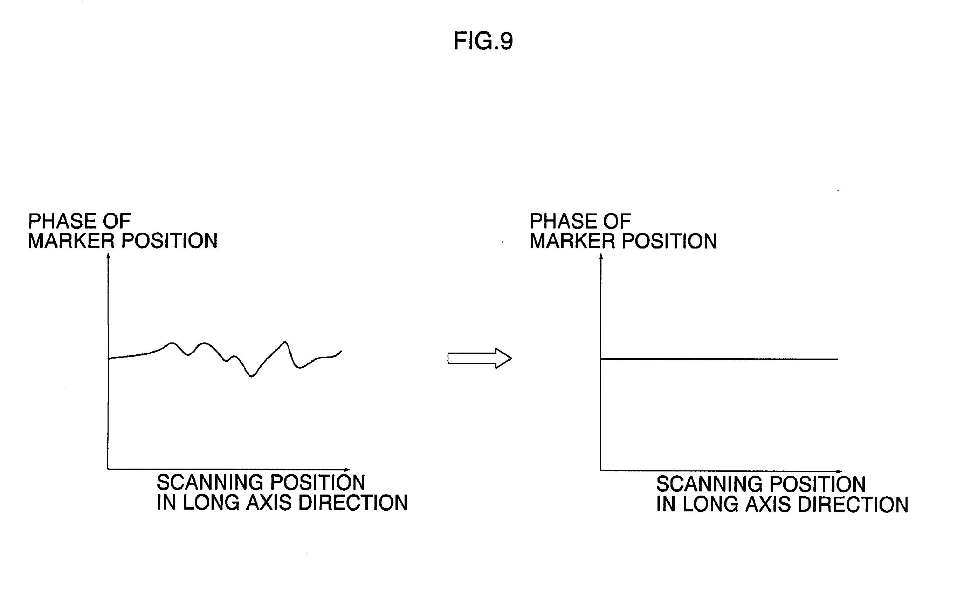

Fig. 9 is a view showing that the optical tomographic images are rotated to match the phase of the marker portion 648. The horizontal axis shows the long axis scanning direction of the probe outer tube 620, and the vertical axis shows the phases of the marker portions 648 and 649. As shown in the figure, even if the phases of the images in the rotation direction are not constant, the phases can be matched by rotating the images and aligning the center portions of the marker portions 648 and 649.

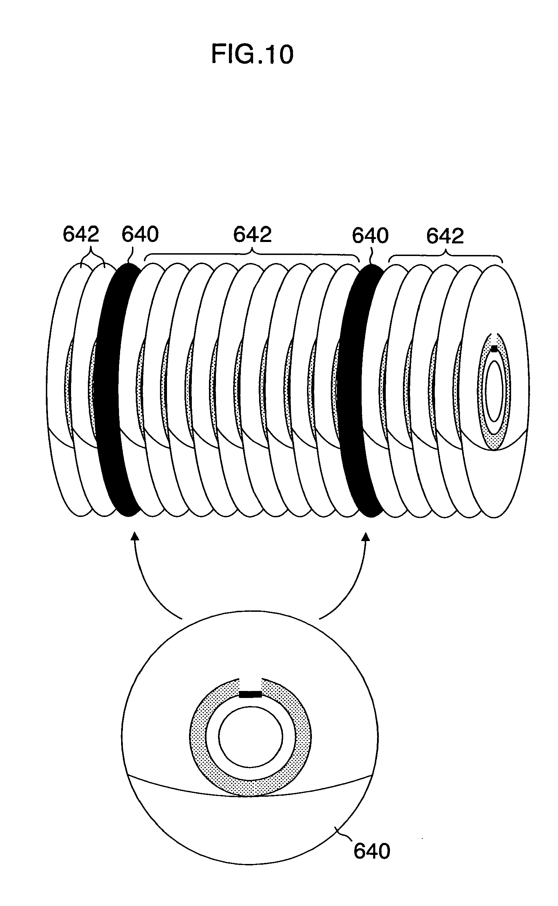

For the plurality of optical tomographic images in which the phases are made uniform in this manner, the interval between the optical tomographic images may not be the same due to the rotation variations of the motor 660, and the like. To correct this, the images 640 in which the marker portion 648 of the movement variations correction marker 630 is present are extracted from the plurality of optical tomographic images (step S3). Fig. 10 is a view showing a state in which the images 640 are extracted. Further, the number of the optical tomographic images 642, which are obtained at positions where the movement variations correction marker 630 is not present, between the extracted images 640 is increased or decreased to be constant (step S4). For example, when there are a portion where four images 642 are present between the images 640, and a portion where five images 642 are present between the images 640, one of the five images 642 is removed, or two images 642 are averaged into one, or the like, to make the number of the optical tomographic images constant.

Using as volume data the plurality of optical tomographic images which are subjected to the above processing, a three-dimensional image is generated (step S5). Thus, the phase precision and position precision of the volume data can be enhanced.

The probe outer tube 620 may be devoid of the rotation variations correction marker 632. In this case, the images 640 should be rotated so that the center portions of the marker portions 648 are in phase, and for the images 642 between the images 640, the phases should be matched using the amount of rotation predicted from the amounts of rotation of the respective images 640.

### <Thickness of Movement Variations Correction Marker>

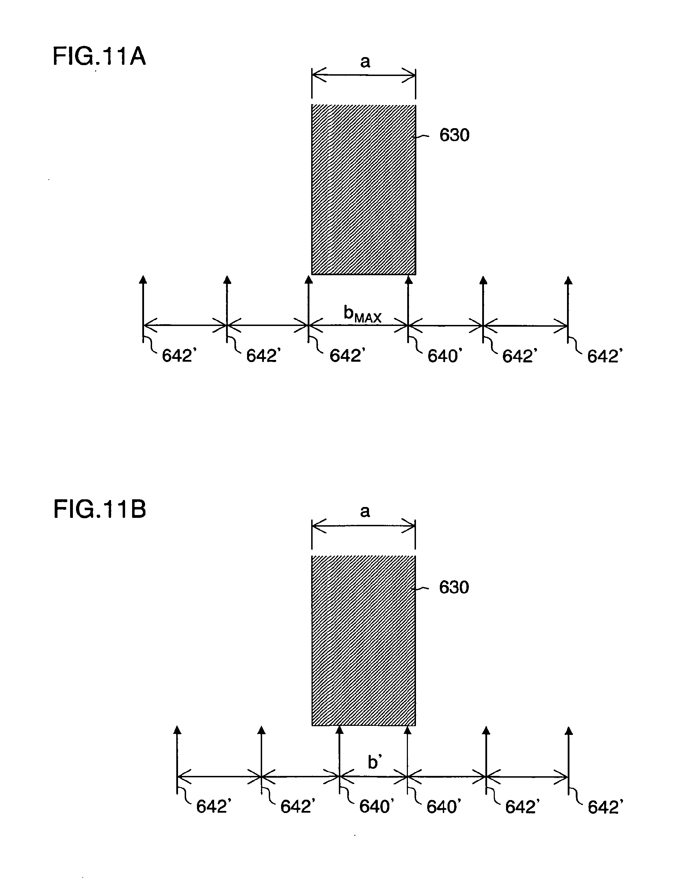

Figs. 11A and 11B are views showing one example of the movement variations correction marker 630 and positions where the image(s) 640 and the images 642 are obtained. Reference numeral 640' designates a position where the image 640 is obtained, and reference numeral 642' designates a position where the image 642 is obtained. It is desired that the thickness a of the movement variations correction marker 630 is thicker than the maximum value of the distance between positions where the optical tomographic images are obtained bₘₐₓ, considering the operation variations of the drive portion, as shown in Fig. 11A. Such thickness can prevent the optical tomographic image from being obtained across the movement variations correction marker 630. In other words, the image 640 can be obtained for all the movement variations correction markers 630.

Also, when the thickness a of the movement variations correction marker 630 is thicker than bₘₐₓ, two continuous optical tomographic images can be obtained at the position of the movement variations correction marker 630, as shown in Fig. 11B. When two continuous images 640 are obtained in this manner, volume data should be generated recognizing that these are at the same marker 630 and that the image 642 is not present between them.

Next, the flow of diagnostic processing will be described.

The diagnostic imaging processing of large intestine pits is performed based on three-dimensional image data created by the above observation and image processing.

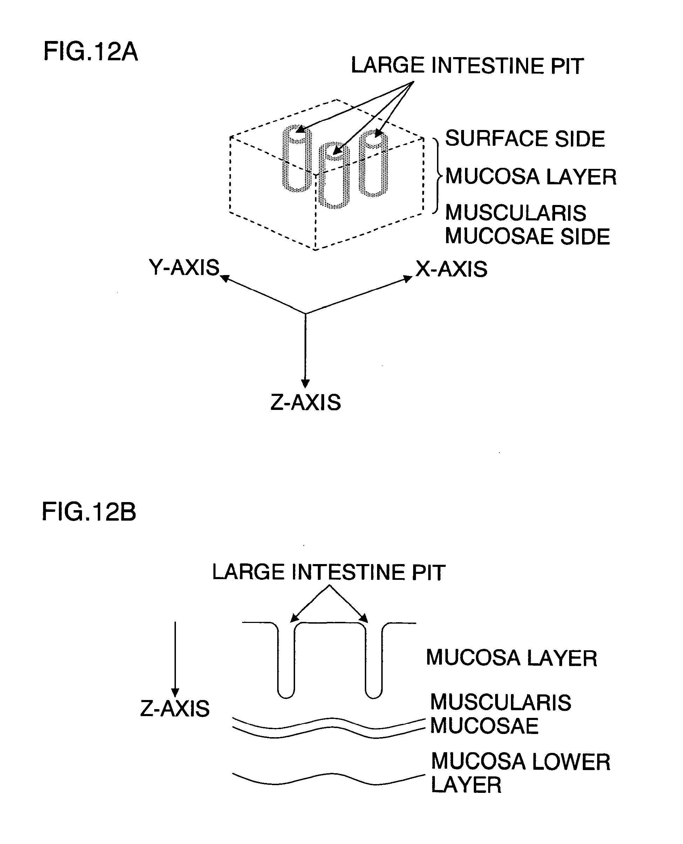

Fig. 12A shows one example of a three-dimensional tomographic image of a large intestine mucosa obtained by OCT and shows a case where it is displayed in the manner of a bird's eye view, with the transmission of each pixel. In the figure, the image is displayed with the density increased according to the intensity of return light.

In Fig. 12A, the Z-axis is the depth direction of the large intestine mucosa, and the X-axis and the Y-axis are set in a plane perpendicular to the Z-axis.

Here, a cross section of one normal large intestine pit is schematically shown in Fig. 12B.

The normal large intestine pit extends cylindrically in the depth direction of the living body tissue (Z-axis).

In the nature of OCT, the return light is intense at the boundary surface between the mucosa and space, so that a state in which the boundary portion between the large intestine pit and space, as a portion where the return light is intense, extends cylindrically in the Z-axis direction, as in the large intestine pit portion in Fig. 12A, is obtained in the three-dimensional tomographic image data.

For the ability of OCT, observation is possible up to a level of a depth of, for example, about 2 mm, and observation is possible up to a position deeper than the muscularis mucosae, so that the entire large intestine pit structure can be grasped.

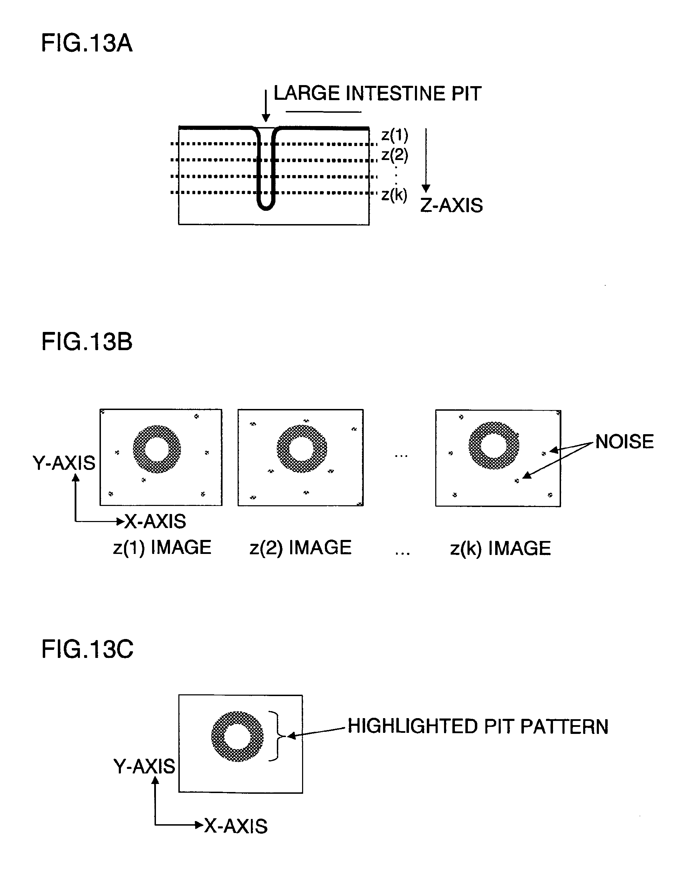

The flow of the diagnostic processing of the large intestine pits will be described with reference to Figs. 13A, 13B, and 13C.

Here, Figs. 13A, 13B, and 13C show a case in a normal large intestine, and show one pit and its peripheral region for simplifying explanation (from the structure of a normal mucosa in Fig. II-1, p. 6, "Large Intestine Pit Pattern Diagnosis" written and edited by Shinei Kudo).

As a first step, an image in an X-Y plane perpendicular to the Z-axis is cut out at predetermined positions in the Z-axis direction (z(1) to z(k)), the depth direction, as in Fig. 13A, in three-dimensional tomographic image data. Here, the depth in the living body tissue increases in the order of z(1) to z(k).

As a second step, the average image of the cut out images in the X-Y planes is created.

In Fig. 13B, the return light signal value in the boundary portion between a pit pattern and space is large, and in the example in the figure, it is displayed in a doughnut shape.

Since the shape of the normal pit is cylindrical, a doughnut-shaped circular shape at substantially the same position and of substantially the same shape is shown in the image in the X-Y plane at each z value.

Here, it is desired that threshold processing is performed. By setting a pixel having a predetermined threshold or less to 0 (a white portion in the figure), noises can be eliminated or reduced.

For example, other than the circular portion of the pit portion, dot-like noises are present in each image in Fig. 13B. They are noises in observation which do not correspond to the original living body tissue, so that the position of their occurrence is random. Therefore, by averaging the plurality of images, as in step 1 and step 2, the effect of noise removal is obtained.

Also, a signal value depending on a living body tissue structure other than the pit is not present over a long distance in the depth direction at a specific position, as in the pit structure, so that when averaged, it is lower than a signal value depending on the pit structure.

On the other hand, the pit pattern at each cut out z position has a high value at a position corresponding to the boundary surface between the tissue and space at the depth. Since the shape of the normal pit is a generally cylindrical shape extending in the Z-axis direction, the shape of the normal pit is also a doughnut-shaped circular shape at substantially the same position and of substantially the same size in each image in the X-Y plane. As a result, a pixel corresponding to the pit pattern has a high value in the average image.

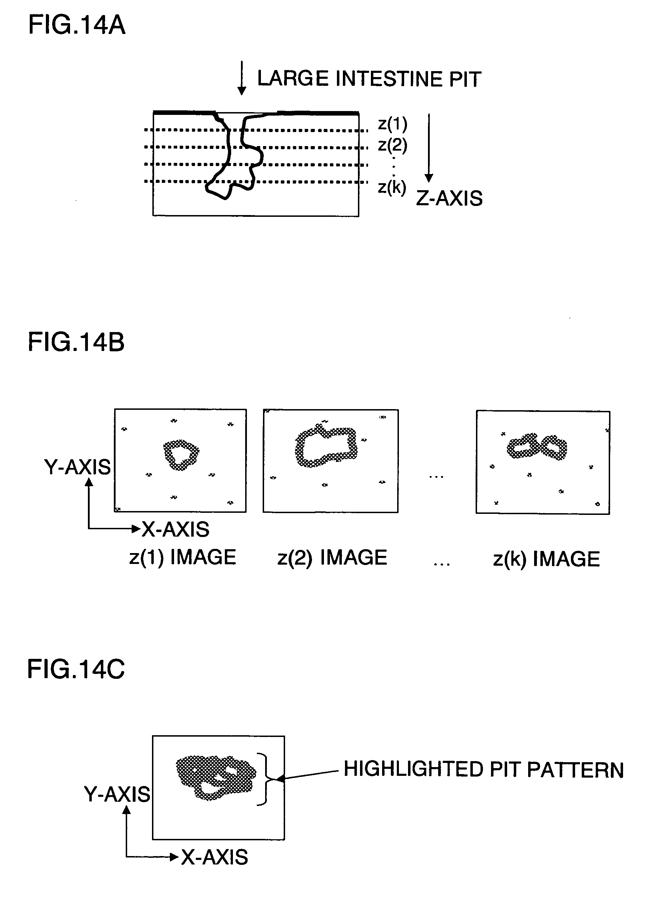

Next, a case of a lesion will be described with reference to Figs. 14A, 14B, and 14C.

In the lesion, the shape of a pit is deformed from the original cylindrical shape, as shown in Fig. 14A.

In this case, in an image in an X-Y plane at each z value in step 1, the shape of the pit that should be a doughnut shape in a normal portion is not a circular shape and is a deformed shape, as shown in Fig. 14B. Therefore, in an average image in step 2, a pixel value in the pit portion is not accumulated on the same pixel, and the value is distributed, so that a clear circular shape is not shown, as shown in Fig. 14C, and the average value is also lower than that of the normal portion.

Diagnosis is performed by displaying the average image on the monitor during diagnosis.

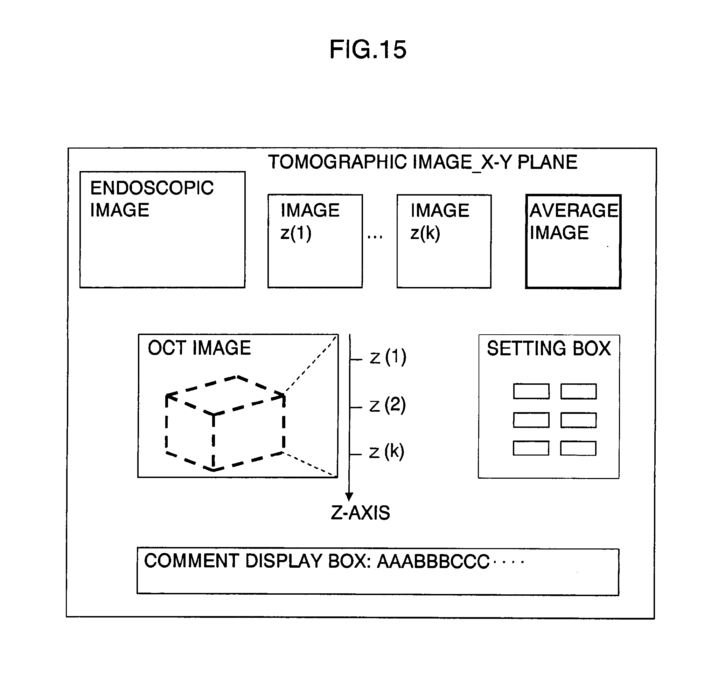

An example of display on a screen for diagnosis is shown in Fig. 15.

For tomographic images, in addition to the display of an average image, images can also be displayed, continuously arrayed so that change in each z value in the depth direction can be observed.

Only one pit is illustrated in Figs. 13A, 13B, and 13C, and Figs. 14A, 14B, and 14C, but it is needless to say that the display area (position and size) in the X-Y plane can be arbitrarily set during diagnosis.

Here, parameters during the creation of the average image can be arbitrarily set.

For example, the range of the value of distance in the depth direction z (the range of z(1) to z(k) in Figs. 13A, 13B, and 13C, and Figs. 14A, 14B, and 14C) can be arbitrarily set. Also, the z value may be continuous pixels or may be pixels at predetermined intervals.

Also, when noise removal is performed by setting a first threshold Th1 or less to 0, the intensity of noise removal may be set by arbitrarily setting this threshold Th1.

Also, a second threshold Th2 or more may be displayed as brightness or color having a predetermined value or more, and this threshold Th2 may be arbitrarily set.

In addition, it is also possible to combine and display a contour map or to perform display by color so that a distribution of signal values in the average image is easily visually recognized.

Next, a second embodiment will be described.

In the second embodiment, for images cut out in X-Y planes in a three-dimensional tomographic image, or their average image in the X-Y plane, an automatic diagnostic point showing a suspicion of a lesion is automatically derived, and is combined with and displayed in an observed image to perform diagnostic support.

Here, the observed image may be a tomographic image (images cut out in X-Y planes or an average image in the X-Y plane) or an endoscopic image.

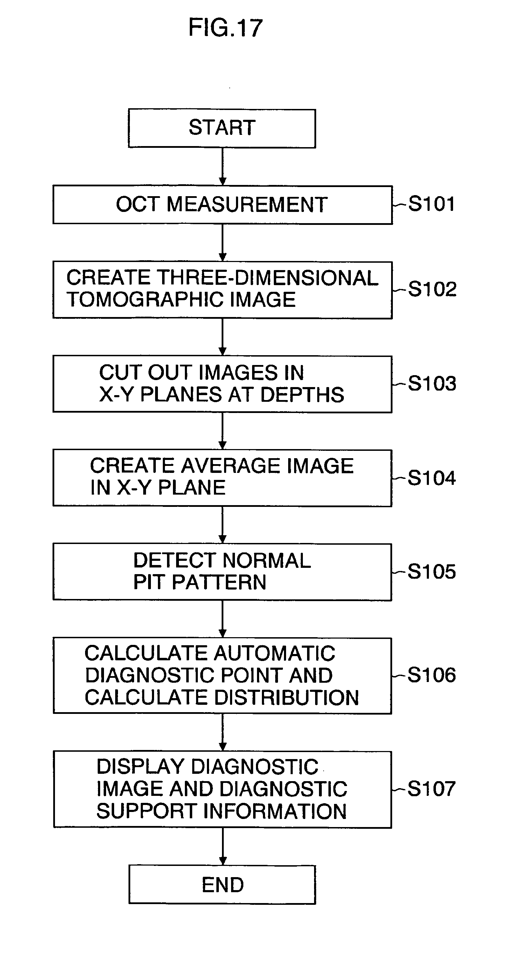

The procedure for creating the average image in the X-Y plane in an observation field of view is similar to that of the first embodiment (s 101 to s 104 in Fig. 17).

First, since an individual normal pit pattern has an annular signal value, the model pattern of the normal pit is set.

Next, the area in the observation field of view is divided into blocks having a predetermined size, and a normal pit pattern having a predetermined similarity or more to the model pattern is detected for each block by a publicly known pattern matching method (s 105).

Then, the summed value of similarity is derived as an automatic diagnostic point for each block, and a distribution in the observation field of view is derived (s 106).

Finally, a contour map is combined with and displayed in the average image in the X-Y plane, based on the summed value for each block, so that the diagnosis of a lesion area can be supported (s107).

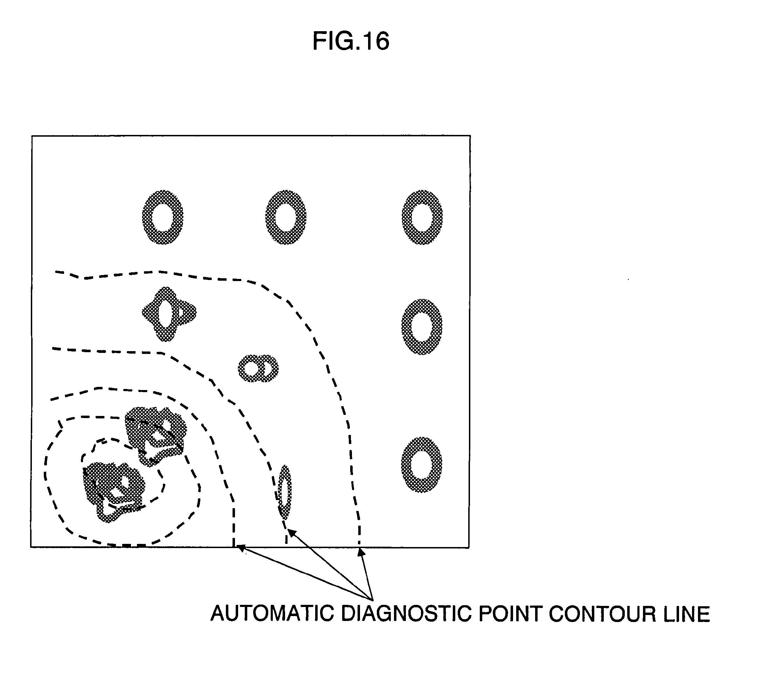

Fig. 16 shows a case where when the similarity value increases as the degree of similarity to a model pattern increases, the automatic diagnostic point is high in the upper area and the right area and decreases toward the lower left area.

The above example is an example of the pattern matching method. In addition, the boundary line of the pit pattern may be obtained by binarization processing or edge detection, and similarity may be derived from the circularity of the boundary line.

For an example of circularity, a publicly known method, such as the ratio of the area inside the boundary line to the length of the boundary line, and the ratio of the width in the x direction to the width in the y direction, should be used.

Alternatively, as another diagnostic support, the difference between the images in the X-Y planes at depths may be scored and displayed. In the case of this method, a possibility that even local shape distortion in the pit not easily discovered in the average image can be sensed increases.

Further, a portion where difference is high may be highlighted in the image in the X-Y plane.

As the images in the X-Y planes at depths, pixels at a specific z value are cut out, but an average image at a specific z value to a predetermined range Δz, that is, at depths of z to (z(i) + range Δz), may be used, wherein i represents an arbitrary position.

In addition, in the above embodiments, a case where the optical probe is a rotating type has been described as the configuration of the OCT apparatus. But, also with a galvano mirror type, three-dimensional tomographic image data can be similarly obtained, so that the processing of the present invention can be performed.

Also, generally, even if a signal obtained by the configuration of the OCT apparatus has unique geometric distortion, an accurate three-dimensional tomographic image can be obtained by applying a publicly known geometric distortion correction method.

Also, in the above embodiments, common three-dimensional coordinates are applied in the observation field of view. But, the area in the observation field of view may be divided into small blocks, and the image in the X-Y plane may be cut out, while, according to the slope of the mucosa surface in the three-dimensional tomographic image in each block, the direction of the Z-axis is finely adjusted to be close to perpendicular to the surface.

The endoscope apparatus of the present invention has been described in detail, but the present invention is not limited to the above examples. Of course, various improvements and modifications may be made without departing from the gist of the present invention.

## Claims

1. A diagnostic imaging apparatus (10), **characterized by** comprising:
a three-dimensional optical tomographic image generating device in which a three-dimensional optical tomographic image is generated, based on return light from a living body obtained by an optical probe (600) inserted into a body to be measured from a forceps port (156) of an endoscope (100),
a setting device in which a Z-axis is set as an axis in a depth direction generally perpendicular to a depth direction of a living body tissue, based on the three-dimensional optical tomographic image data, and an X-axis and a Y-axis are set in a plane perpendicular to the Z-axis, and
a cut out images generating device in which cut out images obtained by cutting out images in X-Y planes at a plurality of levels of depth are generated, so that diagnostic support is performed using the plurality of cut out images.

2. The diagnostic imaging apparatus according to claim 1, wherein
an average image is created by average calculation of pixels in the same coordinates in the X-Y planes, using the plurality of cut out images cut out at the plurality of depths, and
display is performed so that a pit pattern corresponding to a large intestine pit can be observed in the average image.

3. The diagnostic imaging apparatus according to claim 1 and claim 2, wherein a pixel having a predetermined condition is highlighted in the average image.

4. The diagnostic imaging apparatus according to claim 1, wherein the cut out images cut out at the plurality of depths are located so that the cut out images can be compared and observed on the same screen, and display is performed so that a pit pattern corresponding to a large intestine pit can be observed.

5. The diagnostic imaging apparatus according to claim 1 and claim 4, wherein a pixel having a predetermined condition is highlighted in the cut out images.

6. The diagnostic imaging apparatus according to any of claim 1 to claim 5, wherein
a normal large intestine pit pattern is set as a reference pattern,
a pit pattern is extracted from one of the cut out images and the average image,
similarity between the extracted pit pattern and the reference pattern is derived,
an automatic diagnostic point is derived by summation of the similarity for each block which divides an area in an observation field of view into a plurality of areas, and
a distribution of the automatic diagnostic points in the observation field of view is displayed.
